# FASCICULE DE BREVET EUROPEEN

(11) **EP 3 171 825 B1**
(45) Date de publication et mention de la délivrance du brevet: **19.12.2018**
(21) Numéro de dépôt: 15753124.5
(22) Date de dépôt: 24.07.2015
(51) Int. Cl.: A61F 2/38, A61F 2/30

(54) **PROCEDE DE CONCEPTION ASSISTEE PAR ORDINATEUR D'UN IMPLANT SUR MESURE**
COMPUTER ASSISTIERTE METHODE ZUR ENTWICKLUNG EINES PATIENTENSPEZIFISCHEN IMPLANTATS
COMPUTER ASSISTED METHOD FOR DEVELOPPING A PATIENT SPECIFIC IMPLANT

(30) Priorité: 24.07.2014 FR 1457169
(43) Date de publication de la demande: 31.05.2017
(73) Titulaire: One Ortho, 69230 Saint-Genis-Laval (FR)
(72) Inventeur: ALEPEE, Christophe, 69003 Lyon (FR); GUITON, Thierry, 76100 Rouen (FR)
(74) Mandataire: Cabinet Laurent & Charras
(86) Numéro de dépôt international: PCT/FR2015/052051
(87) Numéro de publication internationale: WO 2016/012730

(56) Documents cités:
- WO-A1-2010/099353
- WO-A1-2013/020026
- US-A- 5 735 277
- US-A1- 2007 118 055

## Description

### DOMAINE TECHNIQUE

La présente invention se rapporte au secteur technique de la chirurgie orthopédique, et concerne plus particulièrement un procédé de conception assistée par ordinateur d'un implant sur mesure pour une articulation constituée généralement de deux os d'un patient.

### ETAT ANTERIEUR DE LA TECHNIQUE

Dans la chirurgie orthopédique, il est connu de réaliser des implants sur mesure, notamment au niveau de leur partie fonctionnelle. La partie fonctionnelle de l'implant est adaptée à la morphologie du patient, et permet de reconstruire ou de réparer une zone de l'os détériorée.

Ces implants sur mesure, dont la partie fonctionnelle vise à combler des volumes osseux retirés, donnent satisfaction au niveau du remplissage de l'articulation en tant que telle. Cependant, les parties fonctionnelles osseuses anatomiques présentent des formes qui ne donnent pas un résultat fonctionnel satisfaisant sur le long terme, et peuvent générer des usures majeures et des ruptures mécaniques, faute d'essais et d'expériences cliniques suffisantes. De plus, les parties d'ancrage ne sont pas toujours parfaitement dimensionnées de telle sorte que l'on peut constater des surfaces osseuses non couvertes, source de saignements intempestifs.

Par l'expression « parties d'ancrage » on entend les parties de l'implant destinées à être en contact avec la surface reséquée de l'os, et à réaliser l'ancrage de l'implant dans ledit os. Ces parties d'ancrage comprennent généralement des moyens d'ancrage sous forme de plots, ailettes ou vis, pour l'ancrage en tant que tel. Par l'expression « parties fonctionnelles » on entend les parties de l'implant destinées à coopérer avec des parties complémentaires pour assurer la mobilité et l'articulation en tant que telle.

L'état de la technique pertinent est également divulgué dans les documents WO 2010/099353, WO 2013 020026, US 5,735,277, et US 2007/118055.

### EXPOSE DE L'INVENTION

L'un des buts de l'invention est donc de remédier aux inconvénients précités en proposant un procédé de conception assistée par ordinateur d'un implant sur mesure pour une articulation de deux os d'un patient, qui permette d'optimiser la couverture osseuse au niveau de l'ancrage desdits implants et d'assurer une forme fonctionnelle optimale de l'implant.
Un autre objectif de l'invention est de fournir un procédé qui permette la conception d'un implant parfaitement ajusté au patient au niveau de sa surface de contact et d'appui avec l'os, tout en ayant une partie fonctionnelle standard, sur laquelle un retour clinique satisfaisant est déjà disponible.
La démarche du Demandeur a donc été de revoir l'approche de la conception sur mesure d'implant en permettant de concevoir un implant qui offre, d'une part, des propriétés sur mesure efficaces, et, d'autre part, des performances connues et satisfaisantes bénéficiant d'un retour clinique concret.
A cet effet, il a donc été mis au point un procédé de conception assistée par ordinateur d'un implant sur mesure pour une articulation de deux os d'un patient, le procédé comprenant au moins des étapes consistant à :
- obtenir au moins deux images de l'articulation des deux os ;
- créer un modèle virtuel en trois dimensions d'au moins une portion de l'articulation à partir des images précédemment obtenues.

Conformément à l'invention, le procédé comprend, en outre, des étapes consistant à :
- positionner un volume virtuel d'encombrement prothétique dans l'espace du modèle virtuel, et par rapport à un repère anatomique de référence de ladite articulation, de manière à définir deux zones d'intersection entre ledit volume virtuel d'encombrement prothétique et l'articulation des deux os, lesdites deux zones d'intersection définissant des plans de coupes osseuses nécessaires à la mise en place de l'implant ;
- détecter les contours desdites zones d'intersection ;
- concevoir l'implant sur mesure à partir d'un implant standard, auquel on adapte les contours des surfaces d'appui dudit implant standard, avec les contours préalablement détectés qui correspondent à des préparations osseuses pour une implantation de l' implant;
- fabriquer l'implant conçu.

Ainsi, la surface de contact de l'implant est adaptée d'une manière optimale à la surface de l'os recevant l'implant. La couverture osseuse de l'implant au niveau de son ancrage est optimisée. De cette manière, la charge de l'implant sur la surface de l'os est uniformément répartie, ce qui réduit les contraintes sur l'os et optimise la couverture osseuse pour éviter tout saignement intempestif.

Comme son nom l'indique, le volume d'encombrement prothétique correspond au volume d'encombrement généré par l'empilement des éléments prothétiques de l'implant. Ce volume est compris entre deux plans correspondants aux plans de coupes osseuses à réaliser. Le volume d'encombrement prothétique dépend de l'implant et de l'articulation à corriger. Ce volume est positionné de manière à ce que les zones d'intersection avec les os correspondent auxdits plans de coupes osseuses à réaliser. L'homme du Métier saura, bien entendu, concevoir et positionner correctement ce volume d'encombrement prothétique en fonction de l'articulation concernée et par rapport à un repère anatomique de référence de ladite articulation.

Selon des formes de réalisation particulières, l'implant est conçu à partir d'une bibliothèque virtuelle d'implants pourvus de parties fonctionnelles standards.

Dans une première forme de réalisation, on sélectionne dans la bibliothèque virtuelle un implant comprenant des parties fonctionnelles standards choisies selon la morphologie du patient, et auquel on adapte les contours des surfaces d'appui dudit implant avec les contours détectés.

Dans une deuxième forme, on sélectionne dans ladite bibliothèque, d'une part, des parties fonctionnelles d'implants parmi une pluralité, et, d'autre part, des parties d'ancrage d'implants parmi une pluralité. Les parties d'ancrage sont choisies parmi celles ayant des surfaces d'appui ayant des contours similaires aux contours détectés. L'implant est notamment conçu par une fusion desdites parties fonctionnelles et d'ancrage.

Dans tous les cas, le procédé permet de concevoir un implant sur mesure à partir d'un implant standard, dont les parties fonctionnelles bénéficient déjà d'un retour clinique satisfaisant. L'implant standard est notamment choisi par le praticien chirurgien par exemple, à partir d'un logiciel proposant une palette d'implants standards, et en fonction de la pathologie à traiter.
Avantageusement, on sélectionne également dans ladite bibliothèque virtuelle des moyens d'ancrage parmi une pluralité, tels que plots, ailettes, ou vis. Les moyens d'ancrage sélectionnés sont ménagés sur les surfaces d'appui et de contact de l'implant sur mesure pour l'ancrage en tant que tel.

Un configurateur d'implant sur mesure mis à la disposition d'un utilisateur, par exemple un fabricant d'implant, est également divulgué.

Ce configurateur se présente sous la forme d'un logiciel comprenant une base de données d'implants standards, ou de parties d'implants standards sous la forme d'une bibliothèque d'implants virtuels en trois dimensions.

Un tel configurateur sous la forme d'un produit programme d'ordinateur pour la conception d'un implant sur mesure est également divulgué. Le produit programme d'ordinateur comprend des instructions de code de programme enregistré sur un support utilisable dans un ordinateur.

Ledit produit programme d'ordinateur comprend :
- des moyens de programmation lisibles par ordinateur pour sélectionner un implant standard sur instruction d'un utilisateur, et à partir d'une base de données d'implants standards ;
- des moyens de programmation lisibles par ordinateur pour adapter des contours des surfaces d'appui dudit l'implant, avec des contours résultant d'une intersection entre un volume virtuel d'encombrement prothétique, et un modèle osseux virtuel en trois dimension d'une partie de l'articulation, pour concevoir l'implant sur mesure.
De cette manière, l'invention fournit au fabricant d'implants les moyens de concevoir un implant sur mesure avec l'assistance d'un ordinateur. La conception est simple et intuitive. Le logiciel mis en oeuvre propose d'adapter les parties d'ancrage d'implants standards pour que leurs surfaces d'appui avec l'os comprennent des contours adaptés à des contours préalablement détectés.

Lesdits contours étant préalablement détectés en positionnant un volume virtuel d'encombrement prothétique dans un espace d'un modèle virtuel en trois dimensions de l'articulation, et par rapport à un repère anatomique de référence de ladite articulation, de manière à définir deux zones d'intersection entre ledit volume virtuel d'encombrement prothétique et l'articulation des deux os, lesdites deux zones d'intersection définissant des plans de coupes osseuses nécessaires à la mise en place de l'implant, et lesdits contours préalablement détectés correspondant aux contours des deux zones d'intersection.
Le logiciel permettra par exemple de réaliser l'intersection précitée afin de déterminer automatiquement la forme optimale du contour des surfaces d'appui des implants, ou bien il suffira de renseigner, en tant que données d'entrées du logiciel, les caractéristiques de l'intersection précitée pour définir géométriquement les contours détectés.

Ledit produit programme d'ordinateur peut comprendre:
- des moyens de programmation lisibles par ordinateur pour sélectionner une partie fonctionnelle d'un implant standard sur instruction d'un utilisateur, et à partir d'une base de données de parties fonctionnelles d'implants standards ;
- des moyens de programmation lisibles par ordinateur pour sélectionner des parties d'ancrage d'implants, sur instruction de l'utilisateur, et à partir d'une base de données de parties d'ancrage standards ;
- des moyens de programmation lisibles par ordinateur pour fusionner les parties fonctionnelles et d'ancrage pour concevoir l'implant final sur mesure.
Cette configuration permet de pouvoir concevoir un implant sur mesure à partir de parties d'implants standards, lesquelles bénéficient déjà d'un retour clinique satisfaisant. Cette configuration permet de réaliser des combinaisons d'implants qui n'existent pas sur le marché, et qui sont mieux adaptées aux patients.

Enfin un implant sur mesure pour une articulation de deux os d'un patient et conçu à partir du procédé susmentioné est divulgué.

L' implant comprend une partie fonctionnelle destinée à l'articulation en tant que telle entre les deux os de ladite articulation, et des parties d'ancrage avec l'os. Les parties d'ancrage comprennent des surfaces d'appui dont les contours sont adaptés aux contours des surfaces des os destinées à recevoir l'implant.

Ainsi, l'implant est, sur sa partie destinée à l'ancrage, parfaitement adapté à la morphologie du patient, et standard sur sa partie fonctionnelle pour assurer une efficacité optimale.

L'implant, une fois installé, permet de répartir d'une manière optimale sa charge sur l'os, et diminue ainsi les risques de saignement intempestif.

### BREVE DESCRIPTION DES DESSINS

D'autres caractéristiques et avantages de l'invention ressortiront clairement de la description qui en est réalisée ci-après, à titre indicatif et nullement limitatif, en référence aux figures annexées dans lesquelles :
- la figure 1 est une vue schématique en perspective illustrant l'intersection entre un volume virtuel d'encombrement prothétique et une articulation de deux os d'un patient ;
- la figure 2 est une vue schématique illustrant la couverture osseuse d'un implant de l'état de la technique ;
- la figure 3 est une représentation schématique illustrant la couverture osseuse d'un implant selon l'invention ;
- la figure 4 est une capture d'écran d'un logiciel, selon une forme de réalisation de l'invention, pour illustrer le choix de la forme de l'implant à concevoir ;
- la figure 5 est une capture d'un autre écran du logiciel pour illustrer le choix de l'ancrage de l'implant ;
- la figure 6 est une capture d'un autre écran du logiciel pour illustrer le choix du dimensionnement de l'implant.

### EXPOSE DETAILLE DE L'INVENTION

L'invention concerne un procédé de conception assistée par ordinateur d'un implant (1) sur mesure pour une articulation (2) de deux os d'un patient. L'invention trouve une application pour tout type d'implant d'articulation dans la chirurgie orthopédique, telle que prothèse de genou, prothèse d'épaule, etc. Dans la suite de la description, il sera décrit le cas d'une prothèse de genou.

Pour permettre la conception assistée par ordinateur d'un implant (1) sur mesure, il est nécessaire d'obtenir, en premier lieu, des images médicales de l'articulation concernée. Ces images peuvent être de différents types, telles que celles notamment issues des techniques d'imageries habituelles par exemple IRM, scanner, radiographies, etc.

A partir de ces images médicales, il est créé, par des techniques bien connues de l'homme du métier, un modèle virtuel (3) en trois dimensions d'au moins une partie de l'articulation (2).

Par exemple, lorsque deux radiographies de l'articulation du genou sont réalisées, et selon deux plans orthogonaux, en pratique selon les plans sagittal et frontal, il suffit par exemple pour créer le modèle virtuel (3), de déformer un modèle théorique déjà paramétré, de manière à mettre en coïncidence les deux sections du modèle théorique appartenant aux mêmes plans que les radiographies, avec lesdites deux radiographies. Cette solution permet de « recaler » un modèle virtuel théorique, et de créer un modèle virtuel (3) osseux en trois dimensions de l'articulation (2) concernée.

Dans le cas où l'on dispose d'images médicales en coupes successives selon un axe donné, par exemple issues d'un scanner ou d'un IRM, il suffit pour créer le modèle virtuel (3) de réaliser une reconstruction osseuse en trois dimensions aux moyens d'outils bien connus de l'état de la technique qui permettent, par un traitement des images, et en particulier des niveaux de gris, de définir un contour pour chaque image, puis de placer une surface sur lesdits contours pour générer un solide correspondant au modèle virtuel (3) osseux.

Lorsque le modèle virtuel (3) en trois dimensions de l'articulation (2) du genou est créé à partir de la ou des images précédemment obtenues, on vient positionner selon l'invention, et au moyen d'un logiciel de conception assistée par ordinateur (CAO) par exemple, un volume virtuel d'encombrement prothétique (4) dans l'espace du modèle virtuel (3) osseux.

En effet, lors de l'implantation d'une prothèse, l'implant (1) occupe un certain volume appelé volume d'encombrement prothétique (4). Ce volume est représenté, par exemple, dans le cas d'une prothèse du genou par un polygone, généralement parallélépipédique ou trapézoïdal Dans le cas d'autre prothèse, ce volume peut présenter d'autres formes. La forme du volume d'encombrement prothétique (4) est théorique et n'est pas limitative en tant que telle. Par exemple deux plans parallèles au lieu d'un parallélépipède permettraient de simuler l'encombrement prothétique (4) d'une prothèse de genou.

Pour implanter une prothèse en chirurgie orthopédique, il est nécessaire de veiller à ce que le ou les axes du membre qui contient l'articulation (2) soient correctement alignés, que les coupes des os soient correctement réalisées et positionnées par rapport auxdits axes, et que la balance ligamentaire soit parfaitement ajustée entre les deux coupes pour permettre la stabilité de la prothèse, et la mise en tension stable des ligaments et de la capsule articulaire.

L'implant (1) doit être positionné selon plusieurs repère anatomique de référence, et respecter lesdits rapports anatomiques de référence. Ledit volume d'encombrement prothétique (4) doit donc être positionné de manière à respecter aussi ces rapports anatomiques de référence. Le volume d'encombrement prothétique (4) doit être positionné par rapport à un repère anatomique de référence de ladite articulation (2).

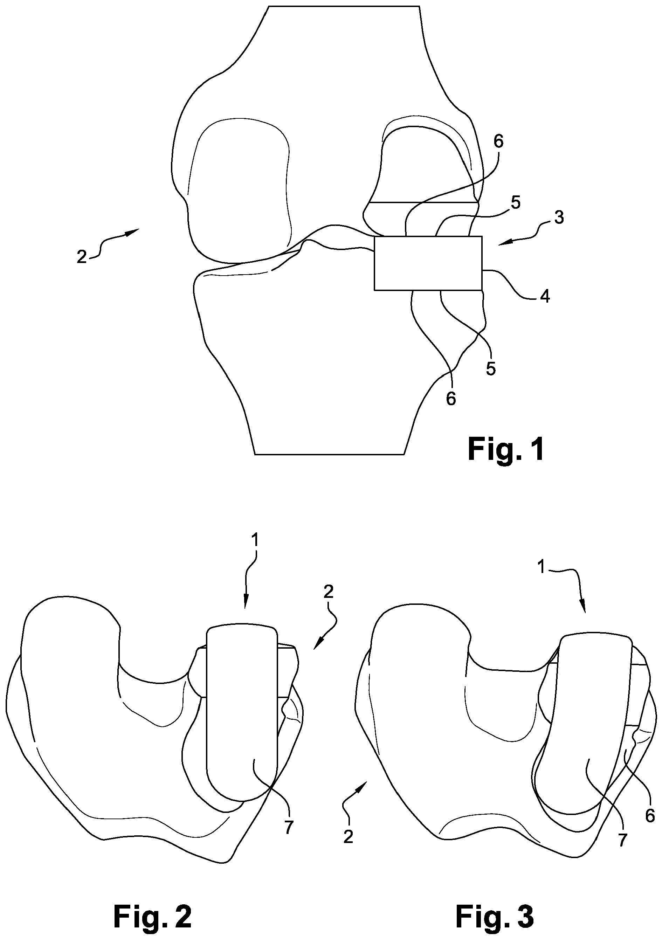

En référence à la figure 1, lorsque le volume d'encombrement prothétique (4) est positionné dans l'espace du modèle virtuel (3) osseux, ceux-ci entrent en intersection de manière à définir deux zones d'intersection (5) entre ledit volume virtuel d'encombrement prothétique (4) et l'articulation (2) des deux os.

Ces deux zones d'intersection (5) correspondent aux surfaces (6) des os qui vont recevoir l'implant (1) en tant que tel. Ces zones d'intersection (5) définissent notamment des plans de coupes ultérieurs pour la mise en place dudit implant (1). Afin de concevoir un implant (1) sur mesure, il convient de réaliser, par exemple par l'intermédiaire du logiciel de CAO, une détection de contours desdites zones d'intersection (5).

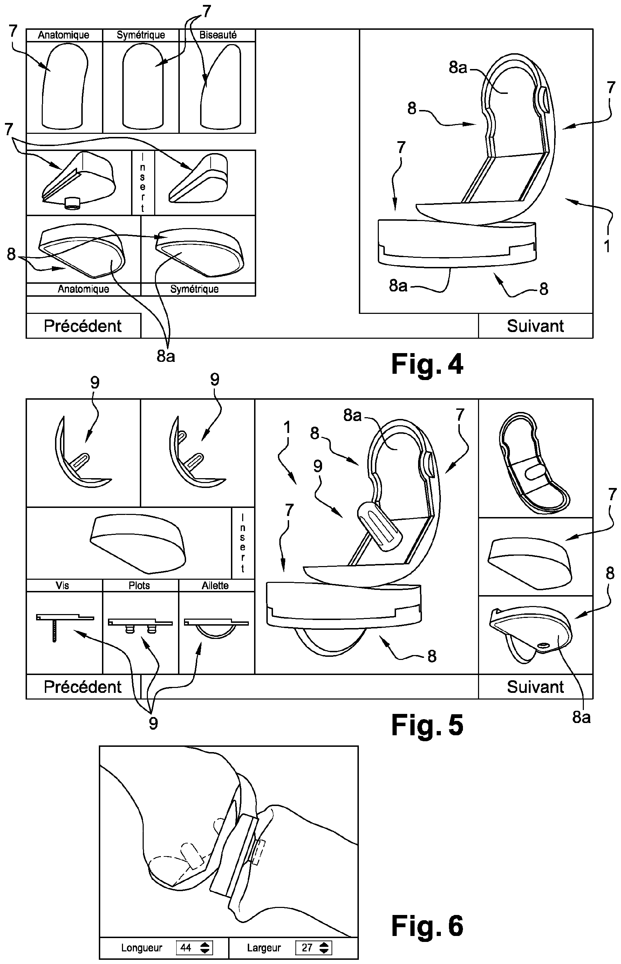

En référence aux figures 4 à 6 qui illustrent des captures d'écran d'un logiciel de conception sur mesure d'un implant, ledit implant (1) comprend, d'une manière générale, des parties fonctionnelles (7) destinées à coopérer avec des parties complémentaires pour assurer la mobilité et l'articulation (2) en tant que telle des deux os du patient, et des parties d'appui et d'ancrage (8) avec les os. Selon l'invention, et en référence à la figure 3, il convient ensuite de concevoir l'implant (1) de manière à ce que, les parties fonctionnelles (7) de l'implant (1) restent standard, et à ce que les surfaces d'appui (8a) dudit implant (1) comprennent des contours adaptés aux contours des zones d'intersection (5) préalablement détectés.

De cette manière, l'implant (1) est sur mesure uniquement sur ses parties d'appui et d'ancrage (8), et est standard sur ses parties fonctionnelles (7). La mise en oeuvre de parties fonctionnelles (7) standards permet d'utiliser des parties fonctionnelles (7) qui ont déjà été utilisées, et qui bénéficient ainsi d'un retour clinique satisfaisant.
Le fait que les parties d'ancrages de l'implant (1) soient sur mesure permet d'assurer une couverture osseuse optimale de l'implant (1). De cette manière, la charge de l'implant (1) sur la surface osseuse est répartie d'une manière optimale, et les saignements intempestifs sont évités.
En référence à la figure 2, un implant de l'état de la technique comprend une partie d'appui et d'ancrage dont la surface d'appui comprend des contours qui ne sont pas adaptés à la surface de l'os qui va recevoir l'implant. Ainsi, selon l'art antérieur, la couverture osseuse n'est pas optimale et il est constaté des saignements intempestifs.

En référence aux figures 4 à 6, 2. un logiciel est fourni par l'intermédiaire duquel le chirurgien choisi un implant standard à modifier pour l'adapter au patient. Ce logiciel présente une bibliothèque virtuelle d'implants (1), ou de parties d'implants (7, 8), standards et en trois dimensions.
Après avoir renseigné le logiciel avec les contours détectés des zones d'intersection (5), ou bien après avoir réalisé ladite détection de contours avec le logiciel, le chirurgien choisi un implant standard adapté à la morphologie à traiter. Par l'intermédiaire du logiciel et en référence à la figure 4, le chirurgien adapte les contours de la surface d'appui (8a) de la partie de contact et d'ancrage (8) de l'implant (1) choisi, avec les contours préalablement détectés.
En référence à la figure 5, le chirurgien peut choisir, avant ou après le dimensionnement de la partie d'ancrage conformément à la figure 6, le type de moyen d'ancrage (9) à agencer sur la surface d'appui (8a) de la partie d'ancrage (8) de l'implant (1) pour réaliser l'ancrage en tant que tel. Le chirurgien peut donc choisir par exemple, des plots, des vis ou des ailettes, ou bien une combinaison de ceux-ci.
Lorsque l'implant (1) est configuré, celui-ci peut être fabriqué par des techniques de fabrications habituelles, telles que par exemple par impression 3D directe ou indirecte.

Le chirurgien peut également concevoir un implant (1) en fusionnant, au moyen du logiciel, des parties fonctionnelles (7) d'un implant standard avec des parties d'ancrage (8) d'un autre implant standard de sorte à concevoir un implant (1) sur mesure.

Comme il ressort de ce qui précède, l'invention fournit un procédé de conception assistée par ordinateur d'un implant (1) sur mesure. La description divulgue aussi les outils pour concevoir l'implant (1), qui permettent la conception d'un implant (1) parfaitement adapté au patient dans sa surface d'appui (8a) avec l'os pour assurer une couverture et une répartition de la charge optimales, et éviter ainsi les risques de saignements intempestifs.

## Revendications

1. Procédé de conception assistée par ordinateur d'un implant (1) sur mesure pour une articulation (2) de deux os d'un patient, le procédé comprenant au moins des étapes consistant à :
- obtenir au moins deux images de l'articulation (2) des deux os ;
- créer un modèle virtuel (3) en trois dimensions d'au moins une portion de l'articulation (2) à partir de la ou des images précédemment obtenues;
- positionner un volume virtuel d'encombrement prothétique (4) dans l'espace du modèle virtuel (3), et par rapport à un repère anatomique de référence de ladite articulation (2), de manière à définir deux zones d'intersection (5) entre ledit volume virtuel d'encombrement prothétique (4) et l'articulation (2) des deux os, lesdites deux zones d'intersection (5) définissant des plans de coupes osseuses nécessaires à la mise en place de l'implant (1);
**caractérisé en ce que** le procédé comprend des étapes consistant à:
- détecter les contours desdites zones d'intersection (5) ;
- concevoir l'implant (1) sur mesure à partir d'un implant standard, auquel on adapte les contours des surfaces d'appui (8a) dudit implant standard avec les contours préalablement détectés qui correspondent à des préparations osseuses pour une implantation de l'implant (1) ;
- fabriquer l'implant (1) conçu.

2. Procédé de conception assistée par ordinateur d'un implant (1) sur mesure selon la revendication 1, ***caractérisé* en ce que** l'implant (1) est conçu à partir d'une bibliothèque virtuelle d'implants pourvus de parties fonctionnelles standards, dans laquelle on sélectionne un implant comprenant des parties fonctionnelles (7) standards désirées, et auquel on adapte les contours des surfaces d'appui (8a) dudit implant avec les contours détectés.

3. Procédé de conception assistée par ordinateur d'un implant (1) sur mesure selon la revendication 1, ***caractérisé* en ce que** l'implant (1) est conçu à partir d'une bibliothèque virtuelle d'implants standards, dans laquelle, on sélectionne, d'une part, des parties fonctionnelles (7) standards désirées d'implants parmi une pluralité, et, d'autre part, des parties d'ancrage (8) d'implants parmi une pluralité, lesdites parties d'ancrage étant choisies parmi celles ayant des surfaces d'appui (8a) ayant des contours similaires aux contours détectés, l'implant (1) étant conçu par fusion desdites parties fonctionnelles (7) et d'ancrage (8).

4. Procédé de conception assistée par ordinateur d'un implant (1) sur mesure selon l'une quelconque des revendications 2 à 3, ***caractérisé* en ce qu'**on sélectionne dans ladite bibliothèque virtuelle des moyens d'ancrage (9) parmi une pluralité, tels que plots, ailettes, ou vis, lesdits moyens d'ancrage (9) sélectionnés étant ménagés sur les surfaces d'appui (8a) de l'implant (1) sur mesure pour l'ancrage en tant que tel.

## Patentansprüche

1. Verfahren zur computerunterstützten Konstruktion eines maßgefertigten Implantats (1) für ein Gelenk (2) aus zwei Knochen eines Patienten, wobei das Verfahren mindestens mehrere Schritte umfasst, die darin bestehen:
- mindestens zwei Aufnahmen des Gelenks (2) aus zwei Knochen zu machen;
- ein dreidimensionales virtuelles Modell (3) mindestens eines Abschnitts des Gelenks (2) ausgehend von den zuvor gemachten Aufnahmen anzufertigen;
- das virtuelle Volumen des prothetischen Platzbedarfs (4) im Raum des virtuellen Modells (3) bezogen auf ein anatomisches Bezugskennzeichen dieses Gelenks (2)zu positionieren, so dass zwei Schnittstellen (5) zwischen diesem virtuellen Volumen des prothetischen Platzbedarfs (4) und des Gelenks (2) aus zwei Knochen definiert werden, wobei die beiden Schnittstellen (5) die Schnittebenen der Knochen definieren, die zum Einsetzen des Implantats (1) notwendig sind;
**dadurch gekennzeichnet, dass** das Verfahren Schritte umfasst, die darin bestehen:
- die Konturen dieser Schnittstellen (5) zu erkennen;
- Konstruktion des maßgefertigten Implantats (1) ausgehend von einem Standardimplantat, wobei die Konturen der Auflagefläche (8a) dieses Standardimplantats mit den zuvor festgestellten Konturen in Übereinstimmung gebracht werden, die der Knochenvorbereitung für die Implantation des Implantats (1) entsprechen:
- Herstellung des konstruierten Implantats (1)

2. Verfahren zur computerunterstützten Konstruktion eines maßgefertigten Implantats (1) gemäß Anspruch 1, **dadurch gekennzeichnet, dass** das Implantat (1) anhand einer virtuellen Implantatbibliothek konstruiert wird, die mit standardmäßigen funktionellen Teilen ausgestattet ist, aus der ein Implantat ausgewählt wird, das die gewünschten standardmäßigen funktionellen Teile (7) aufweist und bei dem die Konturen der Auflageflächen (8a) dieses Implantats an die erkannten Konturen angepasst werden.

3. Verfahren zur computerunterstützten Konstruktion eines maßgefertigten Implantats (1) gemäß Anspruch 1, ***dadurch gekennzeichnet, dass*** das Implantat (1) anhand einer virtuellen Bibliothek mit Standardimplantaten konstruiert wird, aus der einerseits die gewünschten standardmäßigen funktionellen Teilen (7) aus einer Vielzahl und andererseits die Verankerungsteile (8) des Implantats ebenfalls aus einer Vielzahl ausgewählt werden, wobei die Verankerungsteile aus denjenigen ausgewählt werden, die Auflageflächen (8a) haben, deren Konturen ähnlich denen der festgestellten Konturen sind, wobei das Implantat (1) durch Fusion dieser funktionellen (7) und Verankerungsteile (8) konstruiert wird.

4. Verfahren zur computerunterstützten Konstruktion eines maßgefertigten Implantats (1) gemäß Anspruch 2 und 3, ***dadurch gekennzeichnet, dass*** in dieser virtuellen Bibliothek die Verankerungsmittel (9) aus einer Vielzahl ausgewählt werden, wie Plotpunkte, Flügel oder Schrauben, wobei diese ausgewählten Verankerungsmittel (9) in den Auflageflächen (8a) des maßgefertigten Implantats (1) zur Verankerung als solche ausgespart werden.

## Claims

1. Method of computer-aided design of a custom-made implant (1) for a joint (2) of two bones of a patient, the method comprising at least the following steps:
• obtention of at least two images of the joint (2) of the two bones;
• creation of a three-dimensional virtual model (3) of at least one part of the joint (2) from the image(s) previously obtained;
• positioning of a virtual volume of prosthetic space (4) in space of the virtual model (3), relative to an anatomic reference point of said joint (2), so as to define two intersecting areas (5) between said virtual volume of prosthetic space (4) and the joint (2) of the two bones, said two intersecting areas (5) defining planes of bone cross-section necessary for the introduction of an implant (1),
**characterized in that** the method includes the following steps:
• detection of the contours of said intersecting areas (5);
• designing of the custom-made implant (1), from a standard implant, to which one adapts the contours of the bearing surfaces (8a) of said implant with the contours previously detected that correspond to bone preparations for an implantation of the implant (1);
• production of the implant (1) designed.

2. A method of computer-aided design of a custom-made implant (1) in accordance with claim 1, **characterized in that** the implant (1) is designed from a virtual library of implants with standard functional parts, in which one selects an implant with desired standard functional parts (7), and to which one adapts the contours of the bearing surfaces (8a) of said implant with the detected contours.

3. A method of computer-aided design of a custom-made implant (1) in accordance with claim 1, **characterized in that** the implant (1) is designed from a virtual library of standard implants, in which one selects, firstly, desired standard functional parts (7) of implants among a plurality of implants and, secondly, anchoring parts (8) of implants among a plurality of implants, said anchoring parts being chosen from those having bearing surfaces (8a) with contours similar to the detected contours, the implant (1) being designed by fusion of said functional parts (7) and anchoring parts (8).

4. A method of computer-aided design of a custom-made implant (1) in accordance with any one of claims 2 to 3, **characterized in that** one selects in said virtual library anchoring systems (9) among a plurality, such as studs, fins or screws, said selected anchoring parts (9) being positioned on the bearing surfaces (8a) of the custom-made implant (1) for anchoring as such.
